(19) **Europäisches Patentamt**
**European Patent Office**
**Office européen des brevets**

(11) **EP 4 169 447 A1**

(12) **EUROPEAN PATENT APPLICATION**
published in accordance with Art. 153(4) EPC

(43) Date of publication:
26.04.2023 Bulletin 2023/17

(21) Application number: 21858576.8

(22) Date of filing: 18.08.2021

(51) International Patent Classification (IPC):
*A61B 5/35* (2021.01)     *A61B 5/332* (2021.01)
*A61B 5/024* (2006.01)     *A61B 5/00* (2006.01)

(52) Cooperative Patent Classification (CPC):
**A61B 5/00; A61B 5/024; A61B 5/332; A61B 5/35**

(86) International application number:
**PCT/KR2021/010944**

(87) International publication number:
**WO 2022/039489 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 20.08.2020 KR 20200104324

(71) Applicant: **Samsung Electronics Co., Ltd.**
**Gyeonggi-do 16677 (KR)**

(72) Inventor: **JUNG, Hyunjun**
**Suwon-si, Gyeonggi-do 16677 (KR)**

(74) Representative: **Gulde & Partner**
**Patent- und Rechtsanwaltskanzlei mbB**
**Wallstraße 58/59**
**10179 Berlin (DE)**

(54) **METHOD FOR ACQUIRING BIOMETRIC INFORMATION AND ELECTRONIC DEVICE THEREFOR**

(57)     Disclosed is an electronic device comprising: a first biometric sensor configured to acquire pulse wave information; a second biometric sensor configured to acquire biometric information by using a bioelectrical signal; a processor; and a memory. The memory may store instructions that, when executed, cause the processor to: acquire the pulse wave information by using the first biometric sensor; during the acquisition of the pulse wave information, acquire the biometric information corresponding to a plurality of pulse waves by using the second biometric sensor; and acquire a template corresponding to one pulse wave from the biometric information by processing the biometric information by using peak values of the pulse wave information.

FIG.11

# Description

## TECHNICAL FIELD

Various embodiments disclosed in the disclosure relate to a method for acquiring biometric information and an electronic device therefor.

## BACKGROUND ART

Methods for diagnosing a user's health using biometric information such as an electrocardiogram (ECG) are being researched. For example, by observing the ECG waveform, not only temporary abnormal waveforms such as arrhythmia, but also continuous abnormal waveforms may be observed. Continuous abnormal waveforms of the electrocardiogram may appear due to various types of cardiovascular diseases. If it is possible to acquire such an electrocardiogram constantly, diagnosis and prevention of cardiovascular diseases of the user may be quickly performed.

Such electrocardiogram information may be acquired by medical equipment for stable measurements. For example, accurate electrocardiogram information may be acquired by attaching a plurality of skin-attachable electrodes (e.g., wet electrodes) to a subject and performing measurement using medical equipment. In the case of using medical equipment, the subject has to visit a hospital, and it is difficult to measure the electrocardiogram constantly.

## DISCLOSURE OF THE INVENTION

### TECHNICAL PROBLEM

Various user devices having an electrocardiogram measurement function are being released. For example, a watch-type wearable device may be used to measure biometric information such as an electrocardiogram. In general, in the case of using a watch-type wearable device, a user is required to take a specific posture. For example, a specific posture may include a posture in which both hands of the user are in contact with the wearable device. Due to the specific posture, it may be difficult to acquire electrocardiogram information constantly. In addition, electrocardiogram measurement using the wearable device may be vulnerable to noise. Depending on the wearing position of the wearable device, the magnitude of an electrocardiogram signal may be reduced. As the magnitude of the electrocardiogram signal is reduced, vulnerability to noise may occur.

Various embodiments disclosed in the disclosure may provide an electronic device and method for acquiring biometric information such as electrocardiogram information.

## TECHNICAL SOLUTION

According to an aspect of the disclosure, there is provided an electronic device including a first biometric sensor configured to acquire pulse wave information using an optical signal, a second biometric sensor configured to acquire biometric information using a bioelectrical signal, a processor operatively connected to the first biometric sensor and the second biometric sensor, and a memory operatively connected to the processor, and the memory stores instructions that, when executed, cause the processor to acquire heart rate information using the first biometric sensor, acquire the biometric information corresponding to a plurality of pulses using the second biometric sensor, during the acquisition of the pulse wave information, and acquire a template corresponding to one pulse wave from the biometric information by processing the biometric information using peak values of the pulse wave information.

According to another aspect of the disclosure, there is provided a method for acquiring a template of an electronic device; the method including acquiring pulse wave information and biometric information based on a bioelectrical signal, processing the biometric information using peak values of the pulse wave information, and acquiring a template corresponding to one pulse wave of the biometric information using the processed biometric information.

## ADVANTAGEOUS EFFECTS

An electronic device according to an embodiment disclosed in the disclosure may acquire electrocardiogram information with an improved signal-to-noise ratio.

An electronic device according to an embodiment disclosed in the disclosure may provide health information to a user through constant electrocardiogram information acquisition.

Besides, various effects may be provided that are directly or indirectly understood through the disclosure.

## BRIEF DESCRIPTION OF THE DRAWINGS

FIG. 1 is a block diagram illustrating an electronic device in a network environment according to various embodiments.
FIG. 2 illustrates an electrocardiogram measurement environment according to an example.
FIG. 3 illustrates examples of a wearable device.
FIG. 4 illustrates other examples of the wearable device.
FIG. 5 illustrates devices having a function of measuring biometric information.
FIG. 6 illustrates a block diagram of a first electronic device according to an embodiment.

FIG. 7 illustrates a configuration diagram of electronic devices in a case where the first electronic device acquires biometric information from external electronic devices according to an embodiment.

FIG. 8 illustrates a configuration diagram of electronic devices in a case where the first electronic device uses an external electronic device's biometric information and its own biometric information according to an embodiment.

FIG. 9 illustrates synchronization between pieces of biometric information according to an embodiment.

FIG. 10 illustrates acquisition of an electrocardiogram template according to an embodiment.

FIG. 11 illustrates acquisition of an electrocardiogram template according to an embodiment.

FIG. 12 illustrates a heart rate graph and an electrocardiogram graph according to an embodiment.

FIG. 13 illustrates an overlapping graph of electrocardiogram segments and an electrocardiogram template graph according to an embodiment.

FIG. 14 is a flowchart of a method for acquiring a template according to an embodiment.

FIG. 15 is a flowchart corresponding to an operation of acquiring a template according to an embodiment.

With respect to the description of the drawings, the same or similar reference signs may be used for the same or similar elements.

## MODE FOR CARRYING OUT THE INVENTION

Hereinafter, various embodiments disclosed in the disclosure will be described with reference to the accompanying drawings. However, this is not intended to limit the disclosure to the specific embodiments, and it is to be construed to include various modifications, equivalents, and/or alternatives of embodiments of the disclosure.

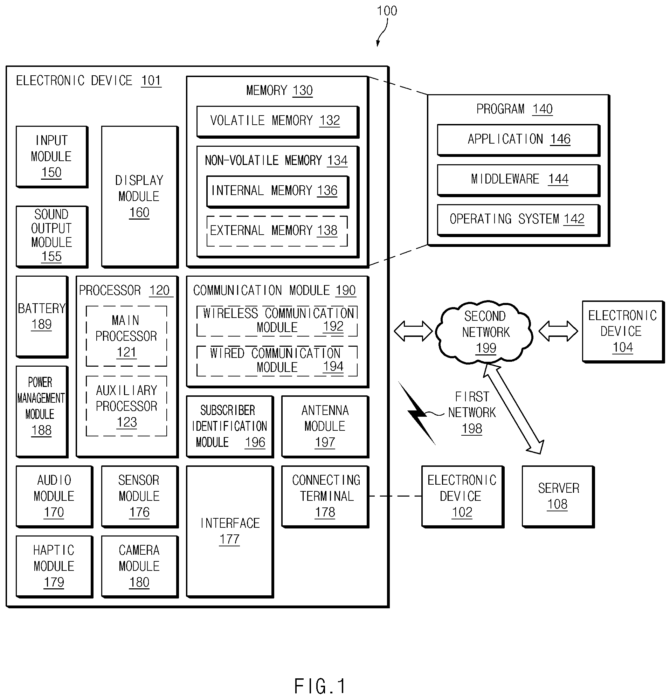

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth™, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN))). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adj acent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play-Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

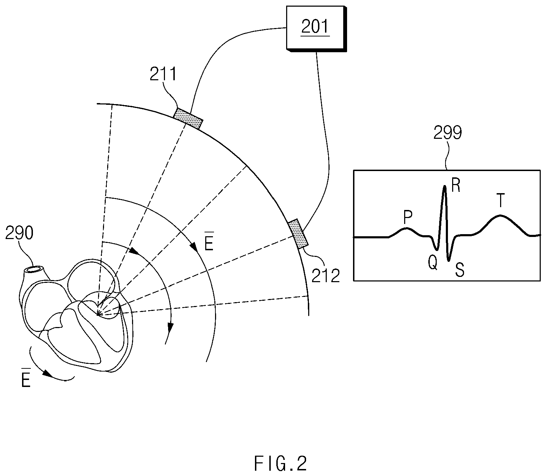

FIG. 2 illustrates an electrocardiogram measurement environment according to an example.

In FIG. 2, an electrocardiogram measuring device 201 may include a first electrode 211 and a second electrode 212 that may contact with a user's skin. In FIG. 2, the first electrode 211 and the second electrode 212 may be positioned in opposite directions with respect to a wearer's heart 290. For example, each of the first electrode 211 and the second electrode 212 may correspond to one of leads 1, 2, or 3 of the Eindhoven triangle. For example, if the electrocardiogram measuring device 201 is a smart watch, the first electrode 211 may come into contact with an arm (e.g., the left arm) on which the smart watch is worn. For example, the first electrode 211 may be positioned on a surface (e.g., a rear surface) of the smart watch that contacts the user's arm when worn. The second electrode 212 may be positioned on a side surface (e.g., bezel) of the smart watch to be able to contact with a finger of an arm (e.g., right arm) on which the smart watch is not worn.

The electrocardiogram measuring device 201 may measure an electric field E (e.g., a potential difference between two specified points (e.g., the position of the first electrode 211 and the position of the second electrode 212) generated by the heartbeat of the heart 290 using the first electrode 211 and the second electrode 212. In FIG. 2, the electrocardiogram measuring device 201 is illustrated as including two electrodes, but the electrocardiogram measuring device 201 may include two or more electrodes for more accurate electrocardiogram measurement.

For example, an electrocardiogram template 299 may be acquired by the electrocardiogram measuring device 201. The electrocardiogram template 299 is an example of an electrocardiogram template possible to be measured in an environment with little noise. The electrocardiogram template 299 may be an electrocardiogram waveform corresponding to one heartbeat. In general, the electrocardiogram template 299 may include a P wave that precedes a QRS complex, the QRS complex, and a T wave that follows. The P wave may appear by depolarization due to contraction of an atrium of the heart 290. The QRS complex may appear by depolarization due to contraction of the ventricles. The T wave may appear by ventricular repolarization due to ventricular relaxation.

The electrocardiogram template 299 may include various health information. Abnormalities of each waveform included in the electrocardiogram template 299 may be derived from various diseases. For example, an abnormal P wave may occur due to an abnormality of the left atrium and/or the right atrium. In addition, the time interval between the respective waveforms may increase and/or decrease due to various diseases. For example, the time between the start point of the P wave and the start point of the QRS complex may increase due to atrioventricular block.

In acquiring an electrocardiogram using a portable electronic device or a wearable electronic device, the position of the electrode may be limited by a form factor (or appearance) of the corresponding electronic device. As shown in FIG. 2, the electrocardiogram measuring device 201 may measure an electrocardiogram by measuring an electromagnetic signal generated by the heart 290. In general, as the measurement position is further away from the heart 290, the magnitude of the electric field E may decrease. For example, the electrocardiogram measuring device 201 may be a device worn at a position distant from the heart 290, such as the neck or head. According to an embodiment, a signal to noise ratio (SNR) may be lowered by decreasing the magnitude of the electric field E. For example, if the measuring is done by the electrocardiogram measuring device 201 worn at a position close to the heart 290, the magnitude of the electrocardiogram signal is about 1 mV, whereas, if the measuring is done by the electrocardiogram measuring device 201 worn at a position distant from the heart 290, the magnitude of the electrocardiogram signal is about 50 μV, which may result in the lowering of the SNR. In addition, the type of electrode (e.g., dry electrode or wet electrode), material constituting the electrode and/or size of the electrode may also be limited by the form factor of the electronic device. For example, the first electrode 211 and the second electrode 212 may be dry electrodes. With the dry electrode, an electrocardiogram signal including a certain level of noise may be acquired. According to an embodiment, as the magnitude of the electric field E decreases, a decrease in SNR may occur with a certain level of noise.

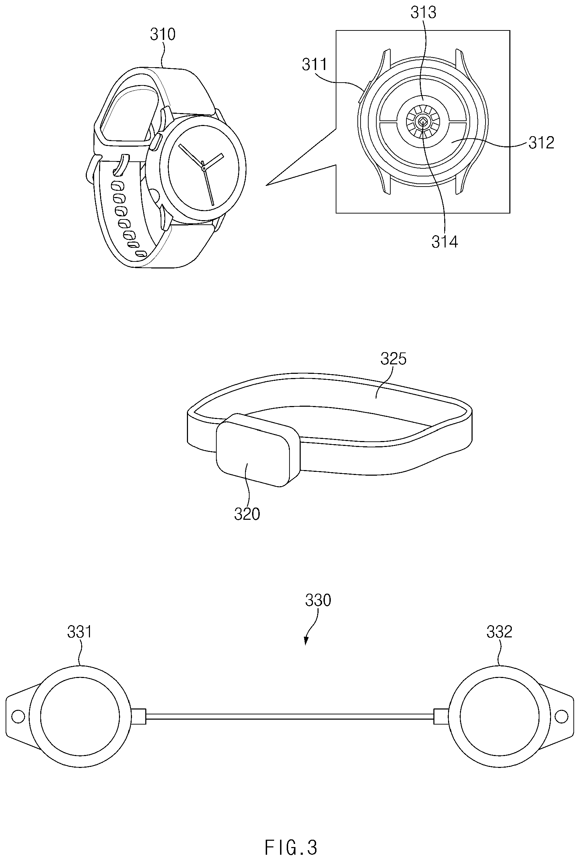

Hereinafter, various electronic devices and methods capable of improving the SNR of biometric information based on bioelectrical signals such as electrocardiograms will be described. In the following examples, the electronic device may acquire an electrocardiogram template using first biometric information and second biometric information. Hereinafter, the first biometric information may include pulse wave information (e.g., heart rate information). The second biometric information may include an electrocardiogram, an electroencephalogram, an electromyogram, an electrooculography (EOG), and/or galvanic skin response (GSR) information. Various types of electronic devices may be described with reference to FIGS. 3 to 5.

FIG. 3 illustrates examples of a wearable device.

For example, the electronic devices of various embodiments disclosed in the disclosure may be a smart watch 310. The smart watch 310 may include an electrocardiogram sensor and/or a heart rate sensor. The smart watch 310 may acquire the second biometric information using an electrocardiogram sensor including a first electrode 311 and a second electrode 312. The smart watch 310 may acquire the first biometric information using the heart rate sensor. For example, the heart rate sensor may include a photoplethysmography (PPG) sensor including at least one light emitting unit 313 and at least one light receiving unit 314. The light emitting unit 313 may include at least one light emitting diode (LED), and the light receiving unit 314 may include at least one photo detector. The heart rate sensor may further include a transimpedence amplifier (TIA), an amplifier circuit, a filter, and an analog-to-digital converter (ADC). In an example, the heart rate sensor may acquire high-quality biometric signals using the light receiving unit 314 including a plurality of photo detector arrays.

The electronic devices according to various embodiments of the disclosure may include a plurality of biometric sensors and may simultaneously acquire the first biometric information and the second biometric information. For example, when a user wears the smart watch 310, an electrocardiogram, an electromyogram, or a GSR may be measured as the user's skin and fingers contact the first electrode 321 and the second electrode 322, and at the same time, the user's heart rate may be measured through the light emitting unit 313 and the light receiving unit 314 of the heart rate sensor that are close to the user's skin.

For example, the electronic devices of various embodiments disclosed in the disclosure may be any sensor device 320. The sensor device 320 may be connected to a band 325 and may be worn on a part of the user's body using the band 325. The sensor device 320 may include at least one sensor for acquiring the first biometric information and/or the second biometric information.

For example, the electronic devices of various embodiments disclosed in the disclosure may be a biometric information measuring device 330. The biometric information measuring device 330 may include a first pad 331 and a second pad 332. For example, the biometric information measuring device 330 may be configured to, while worn on the user, use the first pad 331 and the second pad 332 to acquire the second biometric information about the user (e.g., electrocardiogram, electroencephalogram, EOG, GSR, or electromyogram) based on bioelectrical signals.

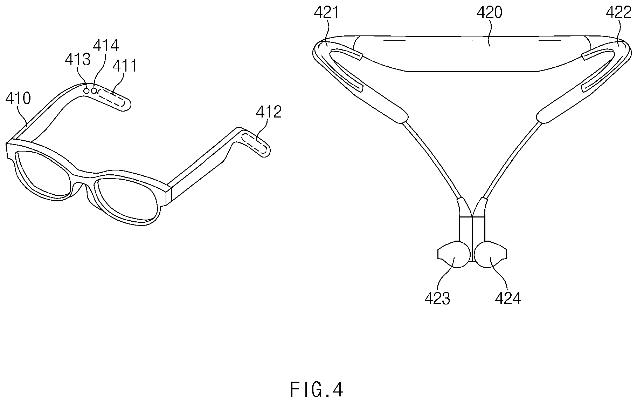

FIG. 4 illustrates other examples of the wearable device.

For example, the electronic devices of various embodiments disclosed in the disclosure may be smart glasses 410. The smart glasses 410 may include a sensor for sensing the first biometric information and/or a sensor for sensing the second biometric information. The smart glasses 410 may acquire the second biometric information by measuring a bioelectrical signal using a first electrode 411 and a second electrode 412. The first electrode 411 and the second electrode 412 may be positioned in opposite directions with respect to a wearer's heart (e.g., the heart 290 of FIG. 2). For example, the sensors of the smart glasses 410 may be positioned on at least a portion of both temples of the smart glasses 410 where the temples may come into contact behind both ears of the wearer when the wearer wears the smart glasses 410. The smart glasses 410 may acquire the first biometric information. For example, the heart rate sensor of the smart glasses 410 may include a photoplethysmography (PPG) sensor including a light emitting unit 413 and a light receiving unit 414. The heart rate sensor of the smart glasses 410 may be positioned on a nose pad 415. The glasses-type wearable device (e.g., the smart glasses 410) may be continuously worn and may sense biometric information such as the first biometric information and/or the second biometric information without the user's separate manipulation while the user is wearing the glasses.

For example, the electronic devices of various embodiments disclosed in the disclosure may be a neckband-type audio device 420. The audio device 420 may include a sensor for acquiring the first biometric information and/or a sensor for acquiring the second biometric information. The audio device 420 may acquire the second biometric information using a sensor including a first electrode 421 and a second electrode 422. The first electrode 421 and the second electrode 422 may be positioned in opposite directions with respect to a wearer's heart (e.g., the heart 290 of FIG. 2). The audio device 420 may acquire the first biometric information using at least one heart rate sensor. For example, the at least one heart rate sensor may include a first heart rate sensor 423 and a second heart rate sensor 424. For example, the first heart rate sensor 423 and the second heart rate sensor 424 may be positioned in an earphone worn on the user's ear.

The electronic devices according to various embodiments of the disclosure may include, for example, at least one biometric information sensor and may simultaneously acquire the first biometric information and the second biometric information. For example, when the user wears the smart glasses 410 or the audio device 420, the second biometric information may be measured using the first electrode 411 or 421 and the second electrode 412 or 422 on the user's skin, and at the same time, pulse wave information about the user may be measured through a sensor close to the user's skin.

With reference to FIGS. 3 and 4, examples of various wearable devices that may be used in the embodiments of the disclosure have been described. However, embodiments of the disclosure are not limited thereto. Any wearable device not disclosed in FIGS. 3 and 4 may be included in electronic devices of various embodiments disclosed in the disclosure. The shape and function of the above-described wearable devices are exemplary, and embodiments of the disclosure are not limited thereto.

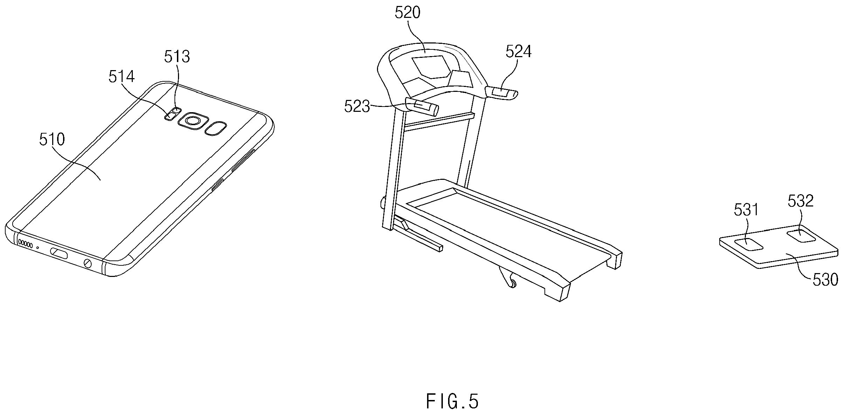

Furthermore, electronic devices of various embodiments disclosed in the disclosure are not limited to the wearable devices. Examples of non-wearable devices corresponding to the electronic devices of various embodiments disclosed in the disclosure may be described with reference to FIG. 5.

FIG. 5 illustrates devices having a function of measuring biometric information.

For example, the electronic devices of various embodiments disclosed in the disclosure may be a mobile phone 510. The mobile phone 510 may acquire the first biometric information using a heart rate sensor. For example, the heart rate sensor may include a PPG sensor including at least one light emitting unit 513 and at least one light receiving unit 514. The mobile phone 510 may acquire the second biometric information using an electrocardiogram sensor. For example, the electrocardiogram sensor may include at least two electrodes (not shown).

For example, the electronic devices in the disclosure may be a treadmill 520. The treadmill 520 may include a first electrode 523 and a second electrode 524. The treadmill 520 may acquire the second biometric information using the first electrode 523 and the second electrode 524. The treadmill 520 may acquire the first biometric information from an external electronic device using a communication circuit.

For example, the electronic devices in the disclosure may be a weight scale 530. The weight scale 530 may include a first electrode 531 and a second electrode 532. The weight scale 530 may acquire the second biometric information using the first electrode 531 and the second electrode 532. The weight scale 530 may acquire the first biometric information from an external electronic device using a communication circuit.

With reference to FIG. 5, examples of various non-wearable devices that may be used in the embodiments of the disclosure have been described. However, embodiments of the disclosure are not limited thereto. Any non-wearable device not disclosed in FIG. 5 may be included in electronic devices of various embodiments disclosed in the disclosure. For example, the mobile phone 510 may include a rollable electronic device or a foldable electronic device. The shape and function of the above-described non-wearable devices are exemplary, and embodiments of the disclosure are not limited thereto.

Electronic devices of various examples have been described with reference to FIGS. 3 to 5. At least some of the electronic devices may not include a biometric sensor for acquiring the first biometric information or the second biometric information, or may have difficulty in simultaneously acquiring the first biometric information and the second biometric information. In this case, the electronic device may acquire insufficient biometric information from other electronic devices. For example, while acquiring the first biometric information using the mobile phone 510, the user may acquire the second biometric information from the treadmill 520 or the weight scale 530 at the same time.

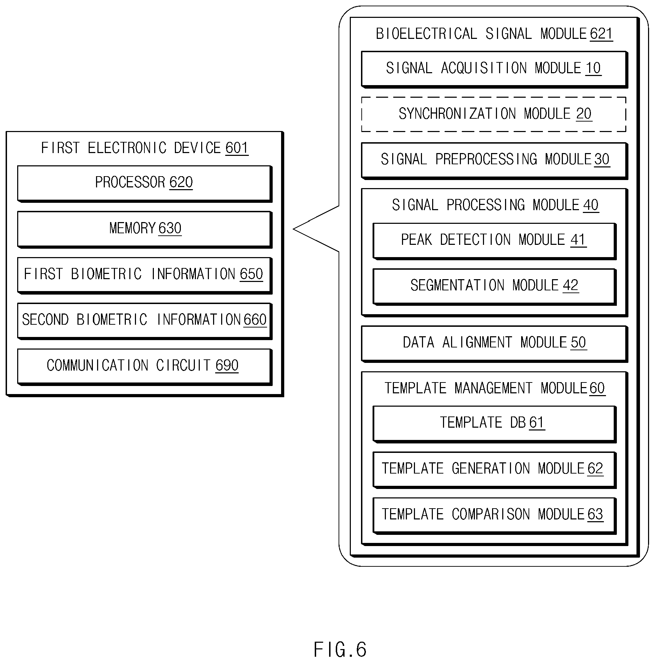

Hereinafter, various electronic devices and systems capable of performing the method for acquiring an electrocardiogram template according to the disclosure may be described with reference to FIGS. 6 to 8. In the following examples, the term "first electronic device" may refer to an electronic device that acquires an "electrocardiogram template" using the first biometric information and second biometric information.

FIG. 6 illustrates a block diagram of a first electronic device according to an embodiment.

According to an embodiment, a first electronic device 601 (e.g., the electronic device 101 of FIG. 1) may include a processor 620 (e.g., the processor 120 of FIG. 1)(e.g., an application processor), a memory 630 (e.g., the memory 130 of FIG. 1), a first biometric sensor 650 (e.g., the sensor module 176 of FIG. 1), a second biometric sensor 660 (e.g., the sensor module 176 of FIG. 1), and/or a communication circuit 690 (e.g., the communication module 190 of FIG. 1). The configuration of the first electronic device 601 illustrated in FIG. 6 is exemplary, and embodiments of the disclosure are not limited thereto. For example, the first electronic device 601 may not include at least one of the components illustrated in FIG. 6. For example, the first electronic device 601 may not include at least one of the first biometric sensor 650 and/or the second biometric sensor 660. For another example, the first electronic device 601 may further include a component not illustrated in FIG. 6.

According to an embodiment, the processor 620 may be operatively or electrically connected to the memory 630, the first biometric sensor 650, the second biometric sensor 660, and/or the communication circuit 690. The processor 620 may control components of the first electronic device 601. For example, the processor 620 may control the components of the first electronic device 601 according to one or more instructions stored in the memory 630. According to an embodiment, the processor 620 may include an application processor.

According to an embodiment, the first biometric sensor 650 may be a sensor configured to acquire the first biometric information (e.g., pulse wave information). The first biometric sensor 650 may include a PPG sensor including at least one light emitting unit and at least one light receiving unit. For example, the first biometric sensor 650 may acquire heart rate information by emitting light using at least one light emitting unit and sensing light received using at least one light receiving unit. According to an embodiment, the at least one light emitting unit may include at least one of a light emitting diode (LED), an organic light emitting diode (OLED), a laser diode (LD), a solid laser, or an infrared diode (IR diode). According to an embodiment, the at least one light receiving unit may include at least one of a photodiode (PD), an avalanche photo diode, a phototransistor, or an image sensor.

According to an embodiment, the second biometric sensor 660 may be a sensor configured to acquire the second biometric information. The second biometric sensor 660 may include an electrocardiogram (ECG) sensor, an electromyogram sensor, an electroencephalogram sensor, an EOG sensor, and/or a GSR sensor, which include a plurality of electrodes. The second biometric sensor 660 may acquire the second biometric information by sensing an electromagnetic signal (e.g., bioelectrical signal) using a plurality of electrodes. The plurality of electrodes may be made of various materials (e.g., metal, indium tin oxide (ITO), and/or conductive polymer). For example, the plurality of electrodes may be positioned at two farthest points within the first electronic device 601 to acquire the maximum electrocardiogram signal.

According to an embodiment, the communication circuit 690 may provide communication with at least one external electronic device. For example, the first electronic device 601 may perform short-range wireless communication using the communication circuit 690. The electronic device 601 may communicate with at least one external electronic device based on near field communication (NFC), Bluetooth, Bluetooth Low Energy (BLE), WiFi Direct, and/or ultra-wideband (UWB) communication. For another example, the first electronic device 601 may perform long-range wireless communication using the communication circuit 690. The first electronic device 601 may access an external network (e.g., a cellular network) using the communication circuit 690.

According to an embodiment, the first electronic device 601 may include a biometric signal module 621. For example, the biometric signal module 621 may be a software module for acquiring a template according to embodiments of the disclosure. Configurations and operations of the biometric signal module 621 may be implemented and performed by the processor 620 and/or the memory 630. For example, the biometric signal module 621 may include a signal acquisition module 10, a synchronization module 20, a signal preprocessing module 30, a signal processing module 40, a data alignment module 50, and/or a template management module 60.

According to an embodiment, the signal acquisition module 10 may acquire the first biometric information and the second biometric information. For example, if the first electronic device 601 includes the first biometric sensor 650 and the second biometric sensor 660, the first electronic device 10 may acquirer the first biometric signal including the first biometric information using the first biometric sensor 650, and may acquire the second biometric signal including the second biometric information using the second biometric sensor 660. For another example, the signal acquisition module 10 may acquire at least one of the first biometric information and/or the second biometric information from an external electronic device. The signal acquisition module 10 may acquire at least one of the first biometric information and/or the second biometric information from at least one external electronic device using short-range wireless communication and/or long-range wireless communication.

According to an embodiment, the synchronization module 20 may synchronize the first biometric information and the second biometric information received from the signal acquisition module 10. For example, if the sampling rates of the first biometric sensor 650 and the second biometric sensor 660 are different or if the first biometric information and the second biometric information are acquired by different devices, the first biometric information and the second biometric information may include data that are not synchronized on a time axis.

The synchronization module 20 may synchronize the first biometric information and the second biometric information, and transmit the synchronized first biometric information and second biometric information to the signal preprocessing module 30. In an embodiment, the synchronization module 20 may be omitted. For example, if sampling rates of the first biometric sensor 650 and the second biometric sensor 660 are the same, or if the first biometric information and the second biometric information are acquired by the same device and the first biometric information and the second biometric information are synchronized, the synchronization module 20 may be omitted.

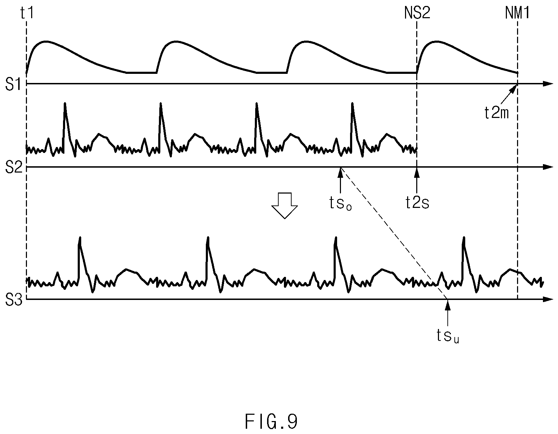

According to an embodiment, the synchronization module 20 may synchronize the second biometric information with the first biometric information based on the sampling rate of the first biometric information. For example, the first biometric information and the second biometric information acquired between time t1 and time t2 may be used. According to an embodiment, the synchronization module 20 may perform synchronization by updating the time stamp ts of the second biometric information using the time stamp of the first biometric information. Specific operations of the synchronization module 20 may be referenced by the description below with reference to FIG. 9.

According to an embodiment, the signal preprocessing module 30 may perform a preprocessing operation on the first biometric information and/or the second biometric information. For example, the signal preprocessing module 30 may perform at least one of preprocessing operations, such as band filtering, noise removal, and/or noise suppression, on the first biometric information and/or the second biometric information. The signal preprocessing module 30 may transmit the preprocessed first biometric information and/or second biometric information to the signal processing module 40.

According to an embodiment, the signal processing module 40 may include a peak detection module 41 and a segmentation module 42. The peak detection module 41 may detect peaks from the first biometric information. The peak detection module 41 may detect peaks using, for example, a maximum value within a specified time window or deconvolution. For example, the peak detection module 41 may be configured to acquire time stamp information corresponding to peak values of the first biometric information. For another example, the peak detection module 41 may be configured to acquire the number of peaks of the first biometric information and the number of peaks of the second biometric information. The segmentation module 42 may segment the second biometric information based on peaks of the first biometric information. If the first biometric information and the second biometric information are synchronized, the segmentation module 42 may generate a plurality of second biometric information segments by segmenting the second biometric information using peak values of the first biometric information. If the first biometric information and the second biometric information are not synchronized, the segmentation module 42 may segment the second biometric information using peak values of the second biometric information.

According to an embodiment, the signal processing module 40 may generate a plurality of second biometric information segments using the unsynchronized first biometric information and second biometric information. For example, the signal processing module 40 may acquire peak values from the first biometric information and the second biometric information acquired during the same time period. The segmentation module 42 may segment the second biometric information using peak values of the second biometric information. After segmentation, the signal processing module 40 may extend or reduce each electrocardiogram segment on the time axis based on the peak values of the first biometric information. Each of the second biometric information segments extended or reduced on the time axis may be synchronized (or aligned) based on the time stamp of the first biometric information. For example, the signal processing module 40 may adjust the second biometric information segments by multiplying each electrocardiogram segment by the ratio of peak values of the first biometric signal to peak values of the second biometric signal. If the number of peak values of the first biometric signal is N1 and the number of peak values of the second biometric signal is N2, which are acquired for the same time, the signal processing module 40 may adjust the scale of the second biometric information segments on the time axis by multiplying the length of the electrocardiogram segment on the time axis by N1/N2. If the interval between peak values of the first biometric signal is PM and the interval between peak values of the second biometric signal is PS, the signal processing module 40 may adjust the lengths of the second biometric information segments on the time axis by multiplying the electrocardiogram segment by PM/PS.

According to an embodiment, the data alignment module 50 may identify a valid second biometric information segment. For example, the data alignment module 50 may exclude exceptional data among a plurality of second biometric information segments and identify the remaining second biometric information segments as valid second biometric data segments. The data alignment module 50 may transmit, to the template management module 60, only close second biometric information segments having a distance (e.g., Euclidean distance) that is equal to or shorter than a specified distance, through principal component analysis of the second biometric information segments. The data alignment module 50 may transmit, to the template management module 60, only the second biometric information segments having deviations (e.g., root mean square (RMS) or amplitude) within a certain range. The data alignment module 50 may transmit, to the template management module 60, only the second biometric information segments having a high degree of correlation, through cross-correlation between the second biometric information segments.

According to an embodiment, the template management module 60 may include a template database (DB) 61, a template generation module 62, and/or a template comparison module 63. The template DB 61 may store a plurality of templates. For example, the template DB 61 may include templates including a specified abnormal waveform (e.g., a plurality of templates corresponding to disease states) and a plurality of templates generated by the template generation module 62. For example, the template may include a waveform of the second biometric information having a length corresponding to one pulse.

According to an embodiment, the template DB 61 may be stored in the memory 630 or stored in an external server (not shown).

According to an embodiment, the template generation module 62 may generate a second biometric information template using the second biometric information segments received from the data alignment module 50. For example, the template generation module 62 may generate a template by averaging the second biometric information segments. The template comparison module 63 may compare a waveform included in a template including an abnormal waveform in the template DB 61 with a waveform included in the generated template. For example, the template comparison module 63 may determine that two waveforms are similar or correspond to each other if the correlation between the two waveforms is equal to or greater than a specified value. For example, if the second biometric information is electrocardiogram information, the template comparison module 63 may determine that the two waveforms are similar or correspond to each other if a Q-T interval and/or a P-R interval between the two waveforms are similar.

If the waveform of the generated ECG template is similar to or corresponds to the abnormal waveform template, the template comparison module 63 may provide, to the user, information on a disease (e.g., heart valve-related disease) corresponding to the specified abnormal waveform. For example, the first electronic device 601 may provide health information (disease information) about the user corresponding to the acquired template through a display (e.g., the display module 160 of FIG. 1), a speaker (e.g., the sound output module 155 of FIG. 1), or a haptic module (e.g., the haptic module 179 of FIG. 1). In an example, the template DB 61 and the template comparison module 63 may be omitted. For example, the template DB 61 and the template comparison module 63 may be implemented by an external server (not shown). The first electronic device 601 may transmit the generated template to an external server using the communication circuit 690.

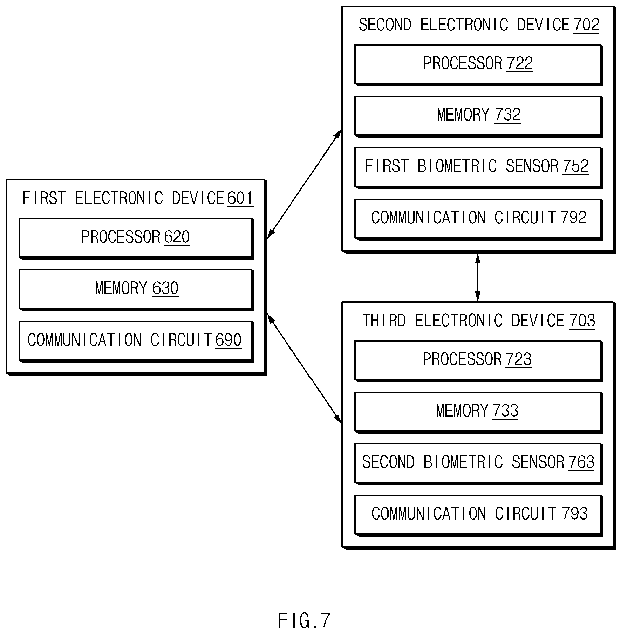

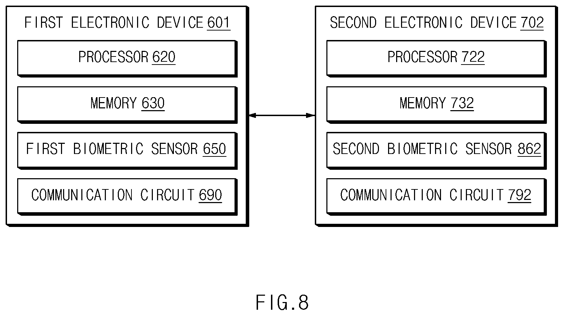

In the example of FIG. 6, it has been described that the first electronic device 601 acquires the first biometric information and/or the second biometric information; however, the embodiments of the disclosure are not limited thereto. For example, as described below with reference to FIGS. 7 and 8, the first electronic device 601 may acquire the first biometric information and/or the second biometric information from an external electronic device.

FIG. 7 illustrates a configuration diagram of electronic devices in a case where the first electronic device acquires biometric information from external electronic devices according to an embodiment.

In the example of FIG. 7, the first electronic device 601 may acquire the first biometric information and the second biometric information from external electronic devices (e.g., a second electronic device 702 and/or a third electronic device 703).

According to an embodiment, a second electronic device 702 (e.g., the electronic device 101 of FIG. 1) may include a processor 722 (e.g., the processor 120 of FIG. 1) (e.g., an application processor), a memory 732 (e.g., the memory 130 of FIG. 1), a first biometric sensor 752 (e.g., the sensor module 176 of FIG. 1), and/or a communication circuit 792 (e.g., the communication module 190 of FIG. 1). The configuration of the second electronic device 702 illustrated in FIG. 7 is exemplary, and embodiments of the disclosure are not limited thereto. For example, the second electronic device 702 may not include at least one of the components illustrated in FIG. 7. For another example, the second electronic device 702 may further include a component not illustrated in FIG. 7.

According to an embodiment, a third electronic device 703 (e.g., the electronic device 101 of FIG. 1) may include a processor 723 (e.g., the processor 120 of FIG. 1) (e.g., an application processor), a memory 733 (e.g., the memory 130 of FIG. 1), a second biometric sensor 763 (e.g., the sensor module 176 of FIG. 1), and/or a communication circuit 793 (e.g., the communication module 190 of FIG. 1). The configuration of the third electronic device 703 illustrated in FIG. 7 is exemplary, and embodiments of the disclosure are not limited thereto. For example, the third electronic device 703 may not include at least one of the components illustrated in FIG. 7. For another example, the third electronic device 703 may further include a component not illustrated in FIG. 7.

Among components of the second electronic device 702 and the third electronic device 703, the description of the components having the same name as the components of the first electronic device 601 of FIG. 6 may be referenced by the description of the components of the first electronic device 601.

According to an embodiment, the first electronic device 601 may be connected to the second electronic device 702 and the third electronic device 703 using the communication circuit 690 through short-range wireless communication (e.g., near field communication (NFC), Bluetooth, Bluetooth Low Energy (BLE), WiFi Direct, and/or ultra-wideband (UWB) communication).

According to an embodiment, the first electronic device 601 may operate as a master device for the second electronic device 702 and the third electronic device 703. According to an embodiment, the second electronic device 702 and the third electronic device 703 may operate as slave devices for the first electronic device 601. The first electronic device 601 may be configured to acquire the first biometric information from the second electronic device 702 and acquire the second biometric information from the third electronic device 703. The first electronic device 601 may generate a template from the first biometric information and the second biometric information using the biometric signal module 621 of FIG. 6. For example, the first electronic device 601 may synchronize the first biometric information and the second biometric information based on its own clock information. The first electronic device 601 may synchronize the first biometric information and the second biometric information based on the time and/or sampling rate of the template of the first electronic device 601. For another example, without synchronizing the first biometric information and the second biometric information, the first electronic device 601 may generate a plurality of second biometric information segments from the second biometric information based on peak values of the second biometric information. The first electronic device 601 may adjust a plurality of second biometric information segments based on a ratio of peak values of the first biometric information to peak values of the second biometric information. The first electronic device 601 may generate a template using at least some of the plurality of second biometric information segments.

According to an embodiment, the first electronic device 601 may operate as a master device for the second electronic device 702 and the third electronic device 703. The first electronic device 601 may acquire the first biometric information and the second biometric information from the second electronic device 702. For example, the first electronic device 601 may not directly communicate with the third electronic device 703, but may communicate through the second electronic device 702. The first electronic device 601 may acquire the first biometric information from the third electronic device 703 through the second electronic device 702.

The connection between the first electronic device 601, the second electronic device 702, and the third electronic device 703 described above with reference to FIG. 7 is exemplary, and the embodiments of the disclosure are not limited thereto. For example, the first electronic device 601 may acquire the first biometric information and the second biometric information from the second electronic device 702 and the third electronic device 703 through an external server (not shown). Further, the second electronic device 702 and the third electronic device 703 may be implemented as one device.

In FIG. 7, it has been described that the first electronic device 601 acquires the first biometric information and the second biometric information from other electronic devices (e.g., the second electronic device 702 and the third electronic device 703); however, as will be described later with reference to FIG. 8, the embodiments of the disclosure are not limited thereto.

FIG. 8 illustrates a configuration diagram of electronic devices in a case where the first electronic device uses an external electronic device's biometric information and its own biometric information according to an embodiment.

In the example of FIG. 8, the first electronic device 601 may acquire the first biometric information using the first biometric sensor 650 and acquire the second biometric information from the second electronic device 702. For example, the second electronic device 702 may acquire the second biometric information using the second biometric sensor 862.

The first electronic device 601 may, for example, synchronize the second biometric information received from the second electronic device 702 with the first biometric information, and generate an electrocardiogram template using the synchronized second biometric information.

In the example of FIG. 8, it has been described that the first electronic device 601 acquires the second biometric information from the second electronic device 702; however, the embodiments of the disclosure are not limited thereto. For example, the first electronic device 601 may include a sensor (e.g., the second biometric sensor 660 of FIG. 6) for acquiring the second biometric information, and may receive the first biometric information from the second electronic device 702.

FIG. 9 illustrates synchronization between pieces of biometric information according to an embodiment.

In FIG. 9, first biometric information S 1 may correspond to heart rate information acquired by the PPG sensor, and second biometric information S2 may correspond to electrocardiogram information acquired by the ECG sensor.

According to an embodiment, a synchronization module (e.g., the synchronization module 20 of FIG. 6) may synchronize the first biometric information S 1 and the second biometric information S2 acquired during the same time period. Since the sampling rates of the first biometric information S 1 and the second biometric information S2 are different, a length NM1 of the first biometric information S 1 and a length NS2 of the second biometric information S2 may be different. Since the sampling rates of the first biometric information S1 and the second biometric information S2 are different, times for measuring the same number of times may be different from each other. Accordingly, when the times are synchronized and visualized, the length NM1 of the first biometric information S1 and the length NS2 of the second biometric information S2 sampled the same number of times (e.g., 4 times) along the time axis may be different. In addition, since there is an error between the clock used to acquire the first biometric information S1 and the clock used to acquire the second biometric information S2, it may be highly likely that the lengths of the first biometric information S1 and the second biometric information S2 acquired for the same time are different. According to an embodiment, the synchronization module 20 may generate synchronized second biometric information S3 by synchronizing the second biometric information S2 based on peak values of the first biometric information S1. For example, the synchronization module 20 may update the time stamp of the second biometric information S2 according to the sampling rate of the first biometric information S1 using Equation 1 below.

[Equation 1]

$$\mathrm{ts_u} = (\mathrm{ts_o} - \mathrm{t1}) * \frac{t2m - t1}{t2s - t1} + \mathrm{t1}$$

In Equation 1 above, $\mathrm{ts_u}$ may represent a time stamp of the synchronized second biometric information S3, and $\mathrm{ts_o}$ may represent a time stamp of the second biometric information S2 before synchronization. In Equation 1 above, t2m may correspond to the time stamp of the first biometric information S1 at time t2, and t2s may correspond to the time stamp of the second biometric information S2 at time t2. The above-described operations of the synchronization module 20 are exemplary, and the synchronization module 20 may generate the synchronized second biometric information S3 by updating the time stamp of the second biometric information S2. The embodiments of the disclosure are not limited thereto. The synchronization module 20 may perform arbitrary operations for synchronizing the first biometric information S1 and the second biometric information S2.

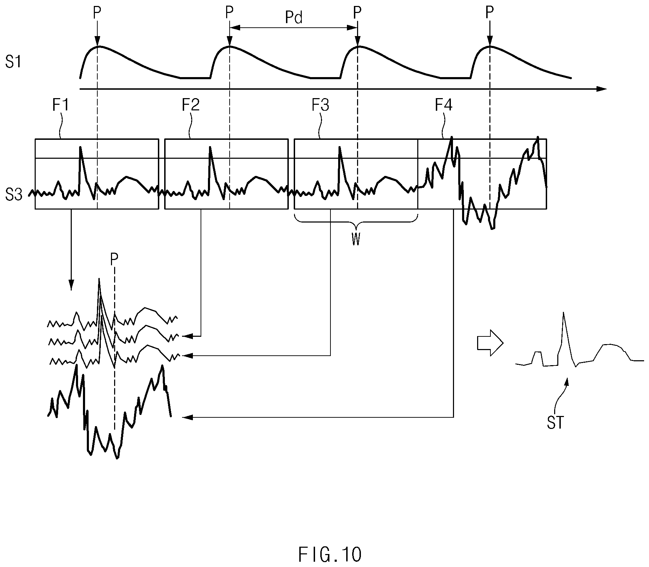

In the example, as described below with reference to FIG. 10, the biometric signal module (e.g., the biometric signal module 621 of FIG. 6) may generate an electrocardiogram template using the second biometric information S3 synchronized with the first biometric information S1.

FIG. 10 illustrates acquisition of an electrocardiogram template according to an embodiment.

In the example of FIG. 10, it may be assumed that the second biometric information S3 synchronized with the first biometric information S1 is synchronized with respect to the same time.

The biometric signal module (e.g., the biometric signal module 621 of FIG. 6) may segment the synchronized second biometric information S3 based on the peaks P of the first biometric information S 1. For example, the biometric signal module 621 may segment the synchronized second biometric information S3 using a window W of a specified length (e.g., one sampling interval) based on the peaks P of the first biometric information S 1. By segmenting the second biometric information S3, the biometric signal module 621 may generate a plurality of electrocardiogram segments F 1, F2, F3, and F4.

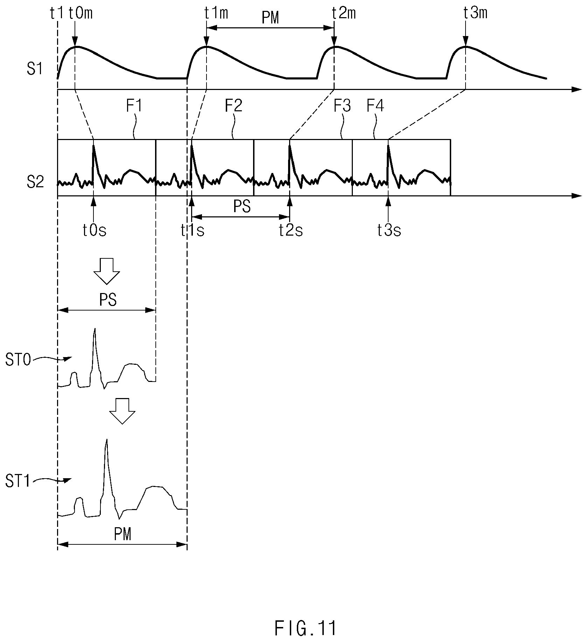

The biometric signal module 621 may align the plurality of electrocardiogram segments F1, F2, F3, and F4. For example, the biometric signal module 621 may align the plurality of electrocardiogram segments F1, F2, F3, and F4 based on the peak P timing (or time stamp). The biometric signal module 621 may identify valid electrocardiogram segments among the plurality of electrocardiogram segments F1, F2, F3, and F4. For example, the biometric signal module 621 may identify valid electrocardiogram segments based on correlation, deviation, and/or similarity. In the example of FIG. 11, the biometric signal module 621 may identify the electrocardiogram segments F1, F2, and F3 as valid electrocardiogram segments, and the electrocardiogram segment F4 as an invalid electrocardiogram segment. The biometric signal module 621 may generate an electrocardiogram template ST using the valid electrocardiogram segments F1, F2, and F3. The biometric signal module 621 may generate the electrocardiogram template ST by averaging the valid electrocardiogram segments F1, F2, and F3.

FIG. 11 illustrates acquisition of an electrocardiogram template.

In FIG. 11, the first biometric information S1 may correspond to heart rate information acquired by the PPG sensor, and the second biometric information S2 may correspond to electrocardiogram information acquired by the ECG sensor. Here, t0m, t1m, t2m, and t3m may indicate peaks of the first biometric information S1, and t0s, t1s, t2s, and t3s may indicate peaks of the second biometric information S2.

In the example of FIG. 11, the peak-to-peak length or the number of peak-to-peak samples of the first biometric information S1 may be PM, and the peak-to-peak length or the number of peak-to-peak samples of the second biometric information S2 may be PS. If synchronization is not performed, the biometric signal module (e.g., the biometric signal module 621 of FIG. 6) may segment the second biometric information S2 into a plurality of electrocardiogram segments F1, F2, F3, and F4 based on the peak values t0s, t1s, t2, and t3s of the second biometric information S2. The biometric signal module 621 may generate an electrocardiogram template ST0 using the electrocardiogram segments and may generate a synchronized electrocardiogram template ST1 by adjusting the size of the electrocardiogram template ST0 by multiplying the generated electrocardiogram template by PM/PS. For example, the biometric signal module 621 may adjust the size of the electrocardiogram template ST0 according to Equation 2 below.

[Equation 2]

$$ST1 = ST0 \times \frac{t2m-t1m}{t2s-t1s}$$

According to an embodiment, the biometric signal module 621 may acquire only PM information from the first biometric information S1 and discard the first biometric information S1, and as a result, the amount of data usage may be reduced.

With reference to FIG. 11, it has been described that the synchronized electrocardiogram template ST1 is acquired after the electrocardiogram template ST0 is acquired; however, the embodiments of the disclosure are not limited thereto. For example, the biometric signal module 621 may generate the synchronized electrocardiogram template ST1 by adjusting the lengths of the electrocardiogram segments F1, F2, F3, and F4 (e.g., adjusting the length by multiplying the electrocardiogram segments by PM/PS) and using at least some of the length-adjusted electrocardiogram segments F1, F2, F3, and F4 to generate an electrocardiogram template.

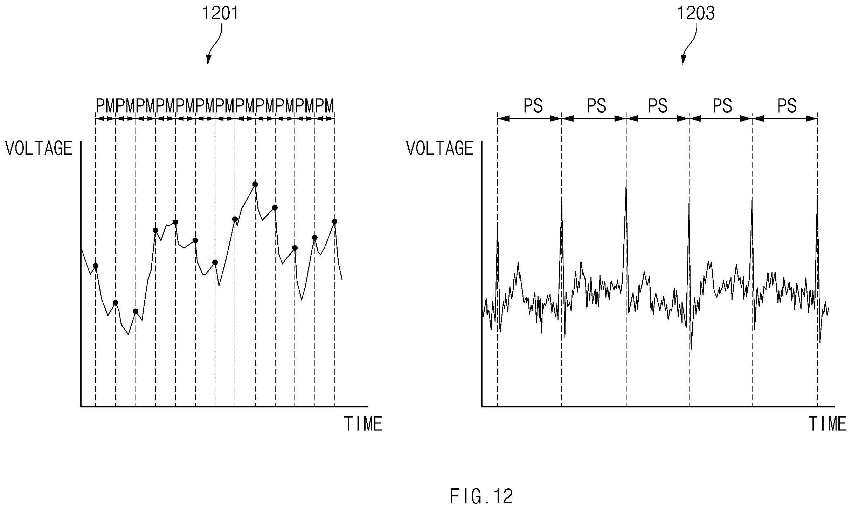

FIG. 12 illustrates a heart rate graph and an electrocardiogram graph according to an embodiment.

A heart rate graph 1201 of FIG. 12 is an example of the first biometric information. The heart rate graph 1201 does not have a constant voltage value on the time axis, but represents peak values at regular intervals (PM). The electrocardiogram graph 1203 of FIG. 12 is an example of the second biometric information. As shown in the electrocardiogram 1203, the QRS complex appears relatively distinctly, but the P wave and T wave may hardly be discerned. By processing the first biometric information and the second biometric information according to the method for acquiring the electrocardiogram template according to the embodiment of the disclosure, a more robust method for acquiring the second biometric information may be provided.

In the example of FIG. 12, the peak values of the second biometric information may have intervals PS, and the peak values of the first biometric information may have intervals PM. For example, as described above with reference to FIGS. 9 to 11, the second biometric information may be segmented based on intervals of peak values.

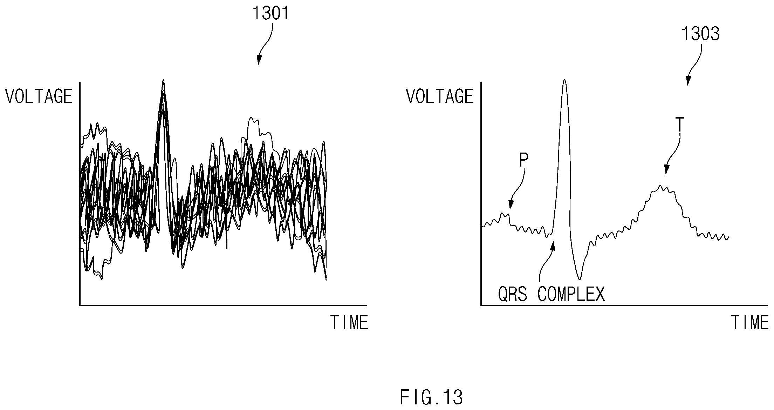

FIG. 13 illustrates an overlapping graph of electrocardiogram segments and an electrocardiogram template graph according to an embodiment.

In an overlapping graph 1301 of electrocardiogram segments of FIG. 13, the electrocardiogram segments aligned based on the same timing may be shown. For example, the overlapping graph 1301 may include electrocardiogram segments having a certain degree of similarity (e.g., correlation value, deviation, and/or distance) among the electrocardiogram segments.

An electrocardiogram template graph 1303 of FIG. 13 may be generated by averaging the electrocardiogram segments of the overlapping graph 1301. In the electrocardiogram template graph 1303, it can be seen that not only QRS complexes, but also the P wave and the T wave appear clearly.

With reference to FIGS. 9 to 13, it has been described that the first biometric information corresponds to the heart rate information and the second biometric information corresponds to the electrocardiogram information; however, the embodiments of the disclosure are not limited thereto.

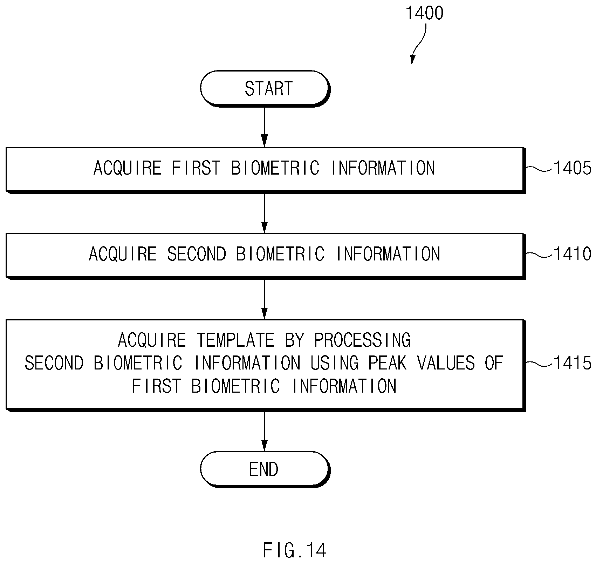

FIG. 14 is a flowchart 1400 of a method for acquiring a template according to an embodiment.

For example, the method for acquiring a template in FIG. 14 may be performed by an electronic device (e.g., the first electronic device 601) described above with reference to FIGS. 6 to 8. Operations of the electronic device to be described later may be performed by a processor (e.g., the processor 620 of FIG. 6) of the electronic device. For example, the processor may be performed when instructions stored in memory (e.g., memory 630 of FIG. 6) are executed.

In operation 1405, the electronic device 601 may acquire first biometric information (e.g., pulse wave information, heart rate information). For example, the electronic device 601 may acquire the first biometric information using a sensor (e.g., the first biometric sensor 650 of FIG. 6) of the electronic device 601. For another example, the electronic device 601 may acquire the first biometric information from an external electronic device (e.g., the second electronic device 702 of FIG. 7).

In operation 1410, the electronic device 601 may acquire second biometric information (e.g., electrocardiogram, electromyogram, electroencephalogram, EOG, or GSR information). For example, the electronic device 601 may acquire the second biometric information based on the bioelectrical signal using a sensor (e.g., the second biometric sensor 660 of FIG. 6) of the electronic device. For another example, the electronic device 601 may acquire the second biometric information from an external electronic device (e.g., the third electronic device 703 of FIG. 7).

In operation 1415, the electronic device 601 may acquire the template by processing the second biometric information using the peak values of the first biometric information. For example, the electronic device may acquire the template using the biometric signal module 621 described above with reference to FIG. 6. According to an embodiment, the electronic device 601 may synchronize the first biometric information and the second biometric information. After synchronizing the first biometric information and the second biometric information, the electronic device 601 may perform preprocessing on the synchronized first biometric information and second biometric information. For example, the electronic device 601 may perform at least one of preprocessing operations, such as band filtering, noise removal, and/or noise suppression, on the first biometric information and/or the second biometric information.

According to an embodiment, the electronic device 601 may detect peaks of the first biometric information and the second biometric information, and segment the second biometric information based on the peaks. For example, the electronic device 601 may segment the second biometric information based on peaks of the first biometric information. The electronic device 601 may generate the template using the segmented second biometric information. For example, the electronic device 601 may acquire the template by averaging the second biometric information segments. The electronic device 601 may acquire the template according to various methods described above with reference to FIGS. 6 to 13.

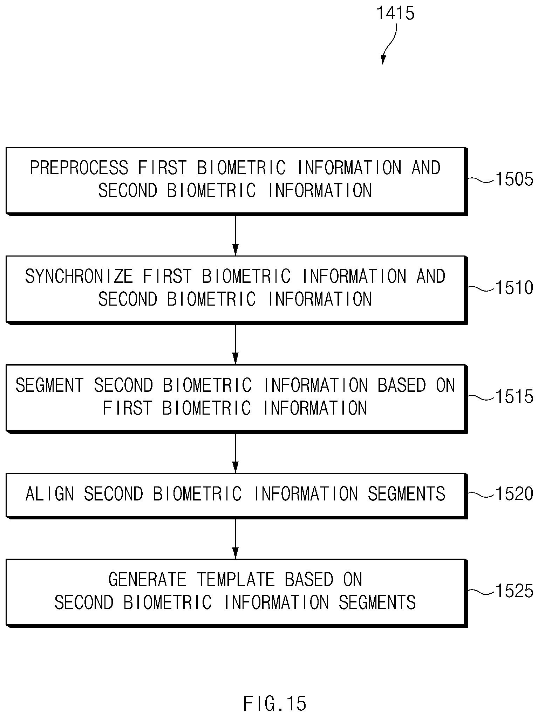

FIG. 15 is a flowchart corresponding to an operation (operation 1415 of FIG. 14) of acquiring a template according to an embodiment. According to various embodiments, at least one operation, but more than the operations shown in FIG. 15 or less than those shown in FIG. 15 may be performed. Operations of the electronic device to be described later may be performed by a processor (e.g., the processor 620 of FIG. 6) of the electronic device. For example, the processor may be performed when instructions stored in memory (e.g., memory 630 of FIG. 6) are executed.

In operation 1505, the electronic device 601 may pre-process the first biometric information and the second biometric information. For example, the electronic device 601 may perform at least one of band filtering, noise removal, and/or noise suppression, on the first biometric information and/or the second biometric information.

In operation 1510, the electronic device 601 may synchronize the first biometric information and the second biometric information. For example, the electronic device 601 may synchronize the second biometric information with the first biometric information in time based on the peak values of the first biometric information. As described above with reference to FIG. 6, the electronic device 601 may not synchronize the first biometric information and the second biometric information. According to an embodiment, operation 1510 may be omitted.

In operation 1515, the electronic device 601 may segment the second biometric information based on the first biometric information. For example, the electronic device 601 may identify peak values of the first biometric information and segment the second biometric information based on the peak values. The electronic device may segment the second biometric information by applying a window having a specified length based on the peak timing to the second biometric information.

In operation 1520, the electronic device 601 may align second biometric information segments. The electronic device 601 may identify valid segments among the second biometric information segments. The electronic device 601 may align the second biometric information segments by identifying valid segments based on similarity (e.g., correlation, distance, and/or deviation) and discarding invalid segments.

In operation 1525, the electronic device 601 may generate the template based on the second biometric information segments. For example, the electronic device 601 may generate the template by averaging the aligned second biometric information segments.

According to an embodiment, the electronic device 601 may store the generated template in a memory (e.g., the memory 630 of FIG. 6). The electronic device 601 may acquire health information about the user by comparing the template with the template including the abnormal waveform stored in a memory.

According to an embodiment, an electronic device (e.g., the first electronic device 601 of FIG. 6 ) may include a first biometric sensor (e.g., the first biometric sensor 650 of FIG. 6) configured to acquire pulse wave information using an optical signal, a second biometric sensor (e.g., the second biometric sensor 660 of FIG. 6) configured to acquire biometric information using a bioelectrical signal, a processor (e.g., processor 620 of FIG. 6) operatively connected to the first biometric sensor and the second biometric sensor, and a memory (e.g., the memory 630 of FIG. 6) operatively connected to the processor. The memory may store instructions that, when executed, cause the processor to perform operations to be described below. The processor may acquire pulse wave information using the first biometric sensor, and may acquire biometric information corresponding to a plurality of pulses using the second biometric sensor while acquiring the pulse wave information. The processor may be configured to acquire a template corresponding to one pulse from the biometric information by processing the biometric information using peak values of the pulse wave information. For example, the processor may generate a plurality of biometric information segments by segmenting the biometric information based on intervals of peak values of the pulse wave information. The processor may be configured to identify a plurality of valid biometric information segments among a plurality of biometric information segments, and to generate the template using the plurality of valid biometric information segments. The processor may identify the plurality of valid biometric information segments among the plurality of biometric information segments, based on the similarities between the plurality of biometric information segments. For example, the electronic device may include a wearable electronic device. For example, the pulse wave information may include heart rate information, and the biometric information may include an electrocardiogram, an electroencephalogram, an electromyogram, an electrooculography (EOG), or a galvanic skin response (GSR).

According to an embodiment, the processor may acquire the template by averaging the plurality of valid biometric information segments. The processor may be configured to perform at least one of filtering, noise removal, or noise suppression on the pulse wave information and/or the biometric information before processing the biometric information.

According to an embodiment, the processor may compare the template with an abnormal waveform stored in the memory, and provide health information corresponding to the template based on the comparison result. For example, the processor may provide health information through a display of the electronic device.

The method for acquiring a template of an electronic device according to an embodiment may include acquiring pulse wave information and biometric information based on a bioelectrical signal, processing the biometric information using peak values of the pulse wave information, and acquiring a template corresponding to one pulse of the biometric information using the processed biometric information. For example, the acquiring of the pulse wave information and the biometric information may include acquiring the pulse wave information and the biometric information using at least one sensor of the electronic device, or receiving at least one of the pulse wave information and the biometric information from an external electronic device. The method may further include synchronizing the pulse wave information and the biometric information before the processing. The synchronizing may include aligning a time stamp of the pulse wave information and a time stamp of the biometric information. The processing of the biometric information may include generating a plurality of biometric information segments by segmenting the biometric information based on intervals of peak values of the pulse wave information. The method may further include identifying peaks of the pulse wave information by performing deconvolution on the pulse wave information. The processing of the biometric information may further include identifying a plurality of valid biometric information segments among the plurality of biometric information segments, and the acquiring of the template may include generating the template using the plurality of valid biometric information segments. The identifying of the plurality of valid biometric information segments may include identifying the plurality of valid biometric information segments based on similarities between the plurality of biometric information segments. The generating of the template using the plurality of valid biometric information segments may include generating the template by averaging the plurality of valid biometric information segments. The method may further include performing filtering, noise removal, or noise suppression on at least one of the pulse wave information and the biometric information before processing the biometric information. For example, the pulse wave information may include heart rate information, and the biometric information may include an electrocardiogram, an electroencephalogram, an electromyogram, an electrooculography (EOG), or a galvanic skin response (GSR).

**Claims**

1. An electronic device comprising:

a first biometric sensor configured to acquire pulse wave information using an optical signal; a second biometric sensor configured to acquire biometric information using a bioelectrical signal; a processor connected to the first biometric sensor and the second biometric sensor; and a memory connected to the processor, wherein the memory stores instructions that, when executed by the processor, cause the electronic device to:

acquire the pulse wave information using the first biometric sensor; acquire the biometric information corresponding to a plurality of pulse waves using the second biometric sensor, during the acquisition of the pulse wave information; and acquire a template corresponding to one pulse wave from the biometric information by processing the biometric information using peak values of the pulse wave information.

2. The electronic device of claim 1, wherein the instructions, when executed by the processor, cause the electronic device to generate a plurality of biometric information segments by segmenting the biometric information based on intervals of the peak values of the pulse wave information.

3. The electronic device of claim 2, wherein the instructions, when executed by the processor, cause the electronic device to:

identify a plurality of valid biometric information segments among the plurality of biometric information segments; and generate the template using the plurality of valid biometric information segments.

4. The electronic device of claim 3, wherein the instructions, when executed by the processor, cause the electronic device to identify the plurality of valid biometric information segments among the plurality biometric information segments based on similarities between the plurality of biometric information segments.

5. The electronic device of claim 3, wherein the instructions, when executed by the processor, cause the electronic device to acquire the template by averaging the plurality of valid biometric information segments.

6. The electronic device of claim 1, wherein the instructions, when executed by the processor, cause the electronic device to perform at least one of filtering, noise removal, and noise suppression on the pulse wave information and/or the biometric information before processing the biometric information.

7. The electronic device of claim 9, wherein the instructions, when executed by the processor, cause the electronic device to:

compare the template with an ideal waveform stored in the memory; and provide health information corresponding to the template based on a result of the comparison.

8. The electronic device of claim 1, wherein the electronic device includes a wearable electronic device.

9. The electronic device of claim 1, wherein the pulse wave information includes heart rate information, and the biometric information includes an electrocardiogram, an electroencephalogram, an electromyogram, an electrooculography (EOG), and/or a galvanic skin response (GSR).

10. A method for acquiring a template of an electronic device, the method comprising:

acquiring pulse wave information and biometric information based on a bioelectrical signal; processing the biometric information using peak values of the pulse wave information; and acquiring a template corresponding to one pulse wave of the biometric information using the processed biometric information.

11. The method of claim 10, wherein the acquiring of the pulse wave information and the biometric information includes:

acquiring the pulse wave information and the biometric information using at least one sensor of the electronic device; or receiving at least one of the pulse wave information and the biometric information from an external electronic device.

12. The method of claim 11, further comprising synchronizing the pulse wave information and the biometric information before the processing.

13. The method of claim 12, wherein the synchronizing includes aligning a time stamp of the pulse wave information and a time stamp of the biometric information.

14. The method of claim 10, wherein the processing of the biometric information includes generating a plurality of biometric information segments by segmenting the biometric information based on intervals of the peak values of the pulse wave information.

15. The method of claim 14, further comprising identifying peaks of the pulse wave information by performing deconvolution on the pulse wave information.

FIG.1

FIG.2

FIG.3

FIG.4

FIG.5

EP 4 169 447 A1

BIOELECTRICAL SIGNAL MODULE 621

SIGNAL ACQUISITION MODULE 10

SYNCHRONIZATION MODULE 20

SIGNAL PREPROCESSING MODULE 30

SIGNAL PROCESSING MODULE 40

PEAK DETECTION MODULE 41

SEGMENTATION MODULE 42

DATA ALIGNMENT MODULE 50

TEMPLATE MANAGEMENT MODULE 60

TEMPLATE DB 61

TEMPLATE GENERATION MODULE 62

TEMPLATE COMPARISON MODULE 63

FIRST ELECTRONIC DEVICE 601

PROCESSOR 620

MEMORY 630

FIRST BIOMETRIC INFORMATION 650

SECOND BIOMETRIC INFORMATION 660

COMMUNICATION CIRCUIT 690

FIG.6

SECOND ELECTRONIC DEVICE 702

PROCESSOR 722

MEMORY 732

FIRST BIOMETRIC SENSOR 752

COMMUNICATION CIRCUIT 792

FIRST ELECTRONIC DEVICE 601

PROCESSOR 620

MEMORY 630

COMMUNICATION CIRCUIT 690

THIRD ELECTRONIC DEVICE 703

PROCESSOR 723

MEMORY 733

SECOND BIOMETRIC SENSOR 763

COMMUNICATION CIRCUIT 793

FIG.7

FIRST ELECTRONIC DEVICE 601

PROCESSOR 620

MEMORY 630

FIRST BIOMETRIC SENSOR 650

COMMUNICATION CIRCUIT 690

SECOND ELECTRONIC DEVICE 702

PROCESSOR 722

MEMORY 732

SECOND BIOMETRIC SENSOR 862

COMMUNICATION CIRCUIT 792

FIG.8

FIG.9

FIG.10

FIG.11

FIG.12

FIG.13

EP 4 169 447 A1

1400

```
          ┌─────────┐
          │  START  │
          └────┬────┘
               │
               ▼
┌─────────────────────────────────────────┐
│  ACQUIRE FIRST BIOMETRIC INFORMATION     │─── 1405
└─────────────────────┬───────────────────┘
                      │
                      ▼
┌─────────────────────────────────────────┐
│  ACQUIRE SECOND BIOMETRIC INFORMATION    │─── 1410
└─────────────────────┬───────────────────┘
                      │
                      ▼
┌─────────────────────────────────────────┐
│        ACQUIRE TEMPLATE BY PROCESSING    │
│  SECOND BIOMETRIC INFORMATION USING PEAK │─── 1415
│        VALUES OF FIRST BIOMETRIC         │
│              INFORMATION                 │
└─────────────────────┬───────────────────┘
                      │
                      ▼
               ┌─────────┐
               │   END   │
               └─────────┘
```

FIG.14

1415

```
┌─────────────────────────────────────────────────┐
│   PREPROCESS FIRST BIOMETRIC INFORMATION AND     │   ～1505
│       SECOND BIOMETRIC INFORMATION               │
└─────────────────────────────────────────────────┘
                        │
                        ▼
┌─────────────────────────────────────────────────┐
│  SYNCHRONIZE FIRST BIOMETRIC INFORMATION AND     │   ～1510
│       SECOND BIOMETRIC INFORMATION               │
└─────────────────────────────────────────────────┘
                        │
                        ▼
┌─────────────────────────────────────────────────┐
│  SEGMENT SECOND BIOMETRIC INFORMATION BASED ON   │   ～1515
│          FIRST BIOMETRIC INFORMATION             │
└─────────────────────────────────────────────────┘
                        │
                        ▼
┌─────────────────────────────────────────────────┐
│   ALIGN SECOND BIOMETRIC INFORMATION SEGMENTS    │   ～1520
└─────────────────────────────────────────────────┘
                        │
                        ▼
┌─────────────────────────────────────────────────┐
│          GENERATE TEMPLATE BASED ON              │   ～1525
│     SECOND BIOMETRIC INFORMATION SEGMENTS        │
└─────────────────────────────────────────────────┘
```

FIG.15

## INTERNATIONAL SEARCH REPORT

| International application No. |
|---|
| **PCT/KR2021/010944** |

**A. CLASSIFICATION OF SUBJECT MATTER**

**A61B 5/35**(2021.01)i; **A61B 5/332**(2021.01)i; **A61B 5/024**(2006.01)i; **A61B 5/00**(2006.01)i

According to International Patent Classification (IPC) or to both national classification and IPC

**B. FIELDS SEARCHED**

Minimum documentation searched (classification system followed by classification symbols)

A61B 5/35(2021.01); A61B 5/00(2006.01); A61B 5/02(2006.01); A61B 5/021(2006.01); A61B 5/022(2006.01); A61B 5/0402(2006.01); A61B 5/0456(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 맥파(pulse wave), 생체 센서(bio sensor), 피크(peak), 분석(analysis), 템플릿 (templet), 인스트럭션(instruction)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0131306 A (SAMSUNG ELECTRONICS CO., LTD.) 26 November 2019 (2019-11-26) See paragraphs [0003]-[0110]; claims 1-5; and figure 4a. | 1,6-11 |
| Y | | 2-5,12-15 |
| Y | JP 2001-137203 A (NIPPON COLIN CO., LTD.) 22 May 2001 (2001-05-22) See claim 1. | 2-5,14,15 |
| Y | KR 10-2015-0038028 A (NEUROSKY, INC.) 08 April 2015 (2015-04-08) See paragraph [0037]; and claim 9. | 12,13 |
| A | JP 2016-093488 A (TOSHIBA CORP. et al.) 26 May 2016 (2016-05-26) See entire document. | 1-15 |
| A | KR 10-0768586 B1 (LAXTHA) 18 October 2007 (2007-10-18) See entire document. | 1-15 |

☐ Further documents are listed in the continuation of Box C.    ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **08 December 2021** | **08 December 2021** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

**PCT/KR2021/010944**

| Patent document cited in search report | | | Publication date (day/month/year) | Patent family member(s) | | | Publication date (day/month/year) |
|---|---|---|---|---|---|---|---|
| KR | 10-2019-0131306 | A | 26 November 2019 | CN | 112135560 | A | 25 December 2020 |
| | | | | EP | 3569142 | A1 | 20 November 2019 |
| | | | | US | 2019-0350464 | A1 | 21 November 2019 |
| | | | | WO | 2019-221438 | A1 | 21 November 2019 |
| JP | 2001-137203 | A | 22 May 2001 | JP | 3496820 | B2 | 16 February 2004 |
| KR | 10-2015-0038028 | A | 08 April 2015 | CN | 104640498 | A | 20 May 2015 |
| | | | | EP | 2895054 | A1 | 22 July 2015 |
| | | | | JP | 2015-536692 | A | 24 December 2015 |
| | | | | JP | 6097834 | B2 | 15 March 2017 |
| | | | | TW | 201423657 | A | 16 June 2014 |
| | | | | US | 2014-0073969 | A1 | 13 March 2014 |
| | | | | WO | 2014-042845 | A1 | 20 March 2014 |
| JP | 2016-093488 | A | 26 May 2016 | JP | 6647831 | B2 | 14 February 2020 |
| | | | | US | 11058361 | B2 | 13 July 2021 |
| | | | | US | 2016-0128643 | A1 | 12 May 2016 |
| KR | 10-0768586 | B1 | 18 October 2007 | None | | | |

Form PCT/ISA/210 (patent family annex) (July 2019)